# EUROPÄISCHE PATENTSCHRIFT

(11) **EP 3 150 641 B1**
(45) Veröffentlichungstag und Bekanntmachung des Hinweises auf die Patenterteilung: **29.08.2018**
(21) Anmeldenummer: 15187284.3
(22) Anmeldetag: 29.09.2015
(51) Int. Cl.: C08F 2/50, C08F 4/72, C07F 7/30, A61K 6/083

(54) **ACYLGERMANIUM-PHOTOINITIATOREN UND VERFAHREN ZU DEREN HERSTELLUNG**
ACYLGERMANIUM PHOTO INITIATORS AND METHOD FOR THEIR PREPARATION
PHOTO-INITIATEURS D'ACYL-GERMANIUM ET PROCEDE DE FABRICATION CORRESPONDANT

(43) Veröffentlichungstag der Anmeldung: 05.04.2017
(73) Patentinhaber: Ivoclar Vivadent AG, 9494 Schaan (LI)
(72) Erfinder: MOSZNER, Norbert, 9493 Mauren (LI); LAMPARTH, Iris, 9472 Grabs (CH); FISCHER, Urs-Karl, 9320 Arbon (CH); STÜGER, Harald, 8042 Graz (AT); HAAS, Michael, 8045 Graz (AT); GESCHEIDT-DEMNER, Georg, 8020 Graz (AT)
(74) Vertreter: Uexküll & Stolberg

(56) Entgegenhaltungen:
- EP-A1- 1 905 413
- EP-A1- 1 905 415

## Beschreibung

Die vorliegende Erfindung betrifft polymerisierbare Zusammensetzungen, die eine Acylgermanium-Verbindung als Polymerisationsinitiator enthalten. Die Zusammensetzungen eignen sich besonders zur Herstellung von Adhäsiven, Beschichtungen, Zementen, Kompositen, Formteilen, wie Stäben, Platten, Scheiben oder Linsen etc., und insbesondere von Dentalwerkstoffen.

Für die Aushärtung von photopolyreaktionsfähigen Harzen spielt der eingesetzte Photoinitiator eine entscheidende Rolle. Bei Bestrahlung mit UV- oder sichtbarem Licht absorbiert dieser Licht und bildet die polymerisationsauslösenden Spezies. Im Fall der radikalischen Polymerisation handelt es sich dabei um freie Radikale. Basierend auf dem chemischen Mechanismus der Radikalbildung werden die Photoinitiatoren in zwei Klassen eingeteilt.

Norrish-Typ-I-Photoinitiatoren bilden beim Bestrahlen durch eine unimolekulare Bindungsspaltung freie Radikale. Norrish-Typ-II-Photoinitiatoren durchlaufen bei der Bestrahlung eine bimolekulare Reaktion, wobei der Photoinitiator im angeregten Zustand mit einem zweiten Molekül, dem Coinitiator reagiert und sich durch Elektronen- und Protonentransfer die polymerisationsauslösenden Radikale bilden. Für die UV-Lichthärtung werden Typ-I- und Typ-II-Photoinitiatoren eingesetzt, für den sichtbaren Lichtbereich kommen bisher, abgesehen von Bisacyldialkylgermanium-Verbindungen, nahezu ausschliesslich Typ-II-Photoinitiatoren zum Einsatz.

Die UV-Härtung zeichnet sich durch eine hohe Reaktionsgeschwindigkeit aus und wird häufig für die Beschichtungen von unterschiedlichen Substraten wie z.B. Holz, Metall oder Glas eingesetzt. So wird zum Beispiel in EP 1 247 843 A2 ein UV-härtendes Beschichtungsmaterial beschrieben, bei dem Typ-I-Photoinitiatoren wie Diethoxyphenylacetophenon oder Acyl- oder Bisacylphosphinoxide zum Einsatz kommen.

Die WO 01/51533 A1 beschreibt ein UV-härtendes Holzbeschichtungsmaterial bei dem ebenfalls Acylphosphinoxide, α-Hydroxyalkylphenone oder α-Dialkoxyacetophenone als Photoinitiatoren Anwendung finden. Bedingt durch die geringe Wellenlänge des UV-Lichts lassen sich mit der UV-Härtung vor allem transparente Beschichtungen mit geringer Schichtstärke realisieren. Bei starker Einfärbung oder Pigmentierung und größeren Schichtstärken kommt man an die Grenzen der UV-Härtung. Derartige photopolyreaktionsfähigen Harze mit deutlich reduzierter Transparenz härten mit UV-Licht nur unvollständig aus.

Werden größere Durchhärtungstiefen benötigt, wie zum Beispiel bei der Aushärtung von lichthärtenden Dentalfüllungsmaterialien, so wird mit sichtbarem Licht bestrahlt. Das dafür am häufigsten eingesetzte Photoinitiatorsystem ist eine Kombination aus einem α-Diketon mit einem Amincoinitiator, wie sie z.B. in GB 1 408 265 beschrieben ist.

Dentalzusammensetzungen, in denen dieses Photoinitiatorsystem eingesetzt wird, werden z.B. in der US 4,457,818 oder der US 4,525,256 offenbart, wobei vorzugsweise Campherchinon als α-Diketon eingesetzt wird. Campherchinon hat ein Absorptionsmaximum bei einer Wellenlänge von 468 nm. Dadurch zeigt Campherchinon eine starke Gelbfärbung, mit dem Nachteil, dass mit Campherchinon/Amin initiierte Materialien nach der Aushärtung häufig einen Gelbstich aufweisen, da das Initiatorsystem nicht vollständig ausbleicht (N. Moszner, R. Liska, Photoinitiators for direct adhesive restorative materials, In: Basics and Applications of Photopolymerization Reactions, Vol. 1; Fouassier, J.-P., Allonas, X., Eds., Research Signpost, Kerala, 2010, 93-114). Dieses Bleichungsverhalten ist vor allem bei hellen Weisstönen des auspolymerisierten Materials sehr nachteilig. Außerdem besitzen Campherchinon-Amin-Systeme bei Anwendung in sauren Adhäsiven den Nachteil, dass die radikalbildende Aminkomponente protoniert und dadurch für die Radikalbildung teilweise deaktiviert wird.

Die Verwendung von Germaniumverbindungen als Photoinitiatoren ist bekannt. Vor allem Bisacyldialkylgermaniumverbindungen sind effiziente Norrish Typ I Photoinitiatoren für die Aushärtung im Blaulichtbereich (B. Ganster, U. K. Fischer, N. Moszner, R. Liska, New photocleavable structures, Diacylgermanbased photoinitiators for visible light curing, Macromolecules 41 (2008) 2394-2400; N. Moszner, U.K. Fischer, B. Ganster, R. Liska, V. Rheinberger, Benzoyl germanium derivatives as novel visible light photoinitiators for dental materials Dent. Mater. 24 (2008) 901-907; N. Moszner, F. Zeuner, I. Lamparth, U. K. Fischer, Benzoylgermanium derivatives as novel visiblelight photoinitiators for dental composites, Macromol. Mater. Eng. 294 (2009) 877-886).

EP 1 905 413 A1 und EP 1 905 415 A1 offenbaren Mono-, Bis- und Triacylgermaniumverbindungen, die sich als Photoinitiatoren zur Härtung von Dentalwerkstoffen mit sichtbarem Licht eigenen. Ihre Synthese ist aufwendig und erfolgt ausgehend von teuren Dialkylgermaniumdihalogeniden unter Nutzung der Dithianschutzgruppentechnik und säulenchromatographischer Reinigung.

Aus der EP 2 103 297 A1 sind als Photoinitiatoren geeignete Acylgermanium-Verbindungen bekannt, die mehrere Germaniumatome enthalten.

Die WO 2015/067815 A1 offenbart Bis(germyl)ketone der Formel R¹R²R³Ge(CO)GeR⁴R⁵R⁶ und Verfahren zu deren Herstellung. Diese Bis(germyl)ketone sollen sich ebenfalls als Photoinitiatoren für Dentalwerkstoffe eignen.

Der Erfindung liegt die Aufgabe zugrunde, Photoinitiatoren für den sichtbaren Bereich bereitzustellen, die sich durch eine verbesserte Reaktivität und Härtungscharakteristik auszeichnen und die sich insbesondere durch sichtbares Licht im langwelligen Bereich aktivieren lassen. Eine weitere Aufgabe der Erfindung ist die Bereitstellung eines vereinfachten Verfahrens zur Herstellung von Acylgermanen.

Diese Aufgabe wird durch Tetra- bzw. Tetrakis-Acylgermane entsprechend der allgemeinen Formel (I) gelöst:

[RₘAr-(C=O)-]₄-Ge (I)

in der die Variablen die folgenden Bedeutungen haben:
- Ar: ein mono- oder polyzyklischer Kohlenwasserstoffrest mit 6 bis 18 Ringkohlenstoffatomen, der m-fach durch die Gruppe R substituiert sein kann und der ein oder mehrere ringständige Heteroatome enthalten kann, wobei
- m: eine ganze Zahl von 0 bis 6 ist und nicht größer als die Anzahl der substituierbaren Wasserstoffatomen in Ar sein kann,
- R: Halogen, NR¹₂, OH, OSiR²₃, (C=O)R³, CN, NO₂, CF₃, COOR⁴, ein C₁ bis C₂₀-Alkyl-, -Alkenyl-, -Alkoxy- oder -Alkenoxy-Rest ist, der linear, verzweigt oder zyklisch sein kann, der durch ein oder mehrere O-Atome unterbrochen sein kann und der eine radikalisch polymerisierbare Gruppe tragen kann, oder =O, wobei
- R¹ bis R³: unabhängig voneinander jeweils H oder ein linearer oder verzweigter C₁- bis C₁₂-Alkyl-Rest sind und
- R⁴: H, ein linearer oder verzweigter C₁- bis C₁₂-Alkyl-Rest oder SiR⁵₃ ist, wobei
- R⁵: ein linearer oder verzweigter C₁- bis C₁₀-Alkyl-Rest ist.

Wenn mehrere Reste R vorhanden sind (m > 1), können diese verschieden oder vorzugsweise gleich sein. Bevorzugte radikalisch polymerisierbare Gruppen, die bei den Resten R als Substituenten vorhanden sein können, sind Vinyl, Styryl, Acrylat (CH₂=CH-CO-O-), Methacrylat (CH₂=C(CH₃)-CO-O-), Acrylamid (CH₂=CH-CO-NR⁶- mit R⁶ = H oder C₁-C₈-Alkyl), Methacrylamid (CH₂=C(CH₃)-CO-NH-), besonders bevorzugt (Meth)acrylat, Methacrylamid und/oder N-Alkylacrylamid. Vorzugsweise tragen der oder die Reste R 0 bis 3, insbesondere 0 bis 1 radikalisch polymerisierbare Gruppen. Die polymerisierbaren Gruppen sind bei nichtzyklischen Resten vorzugsweise endständig angeordnet.

Verbindungen, in denen Ar eine unsubstituierte Gruppe ist, sind nach den Regeln der chemischen Nomenklatur als Tetraacylgermane zu bezeichnen, während Verbindungen, in denen Ar substituiert ist, als Tetrakis(acyl)germane bezeichnet werden müssen. Der Einfachheit halber wird hier die Bezeichnung Tetraacylgermane für beide Verbindungsgruppen verwendet.

Ar ist vorzugsweise ein polyzyklischer Kohlenwasserstoffrest, der mindestens einen aromatischen Ring enthält, besonders bevorzugt ein aromatischer Kohlenwasserstoffrest. Bevorzugte polyzyklische Kohlenwasserstoffreste mit mindestens einem aromatischen Ring sind Anthrachinon und Naphthochinon. Als aromatische Kohlenwasserstoffreste sind neben dem Benzolrest insbesondere kondensierte aromatische Gruppen wie Naphthalin-, Anthracen-, Phenanthren- und Naphthacengruppen bevorzugt.

Ar kann ein oder mehrere, vorzugsweise 1 bis 2 ringständige Heteroatome enthalten. Bevorzugte Heteroatome sind 0, S und besonders bevorzugt N. Besonders bevorzugte heteroaromatische Reste sind Pyridin, Pyrimidin und Chinolin.

Durch die Formel (I) und die übrigen hierin gezeigten Formeln werden sämtliche stereoisomere Formen sowie Gemische verschiedener stereoisomerer Formen, wie z.B. Racemate, erfasst. Die Formeln erfassen nur solche Verbindungen, die mit der chemischen Valenzlehre vereinbar sind. Beispielsweise kann m nicht größer als die Zahl der substituierbaren Wasserstoffatome der Gruppe Ar sein. Wenn R über zwei Bindungen an Ar gebunden ist, ist die maximale Anzahl möglicher Reste R entsprechend geringer.

Der Hinweis, dass ein Rest durch ein Heteroatom wie 0 unterbrochen sein kann, ist so zu verstehen, dass die O-Atome in die Kohlenstoffkette oder den Kohlenstoffring des Restes eingeschoben werden, d.h. beidseitig von Kohlenstoffatomen begrenzt werden. Die Anzahl der Heteroatome ist daher um mindestens 1 kleiner als die Zahl der Kohlenstoffatome, und die Heteroatome können nicht endständig sein. Bei Kohlenwasserstoffresten, die Kohlenstoff- und Heteroatome enthalten, ist die Anzahl der Heteroatome ohne Berücksichtigung von Substituenten stets kleiner als die Zahl der Kohlenstoffatome.

Halogen (abgekürzt Hal) steht vorzugsweise für F, Cl, Br oder I, insbesondere für F, Cl, ganz besonders bevorzugt für Cl.

Besonders bevorzugt sind Tetraacylgermane entsprechend der allgemeinen Formel (I), bei denen die Variablen die folgenden Bedeutungen haben:
- Ar: ein aromatischer C₆-C₁₀-Rest, der m-fach durch R substituiert sein kann, wobei
- m: eine ganze Zahl von 1 bis 3 ist und
- R: Cl, NR¹₂, OSiR²₃, (C=O)R³, CN, NO₂, CF₃, COOR⁴, oder ein C₁ bis C₁₀-Alkyl-, -Alkenyl-, -Alkoxy- oder -Alkenoxy-Rest ist, der linear, verzweigt oder zyklisch sein kann, der durch ein oder mehrere O-Atome unterbrochen sein kann, und der eine radikalisch polymerisierbare Gruppe, vorzugweise Vinyl, Methacrylat, (Meth)acrylamid oder N-Alkylacrylamid enthalten kann, wobei die radikalisch polymerisierbare Gruppe bei nichtzyklischen Resten vorzugsweise endständig ist, wobei
- R¹ bis R³: unabhängig voneinander jeweils H oder ein linearer oder verzweigter C₁- bis C₈-Alkyl-Rest sind und
- R⁴: H, ein linearer oder verzweigter C₁- bis C₈-Alkyl-Rest oder SiR⁵₃ ist und
- R⁵: ein linearer oder verzweigter C₁- bis C₅-Alkyl-Rest ist.

Weiter bevorzugt sind Tetraacylgermane entsprechend der allgemeinen Formel (I), bei denen die Variablen die folgenden Bedeutungen haben:
- Ar: ein Phenyl-Rest, Pyridyl-Rest, Naphthyl-Rest, Anthryl-Rest, Anthrachinonyl-Rest, der m-fach durch R substituiert sein kann, wobei
- m: eine ganze Zahl von 1 bis 3 ist und
- R: NR¹₂, CN, NO₂, CF₃, ein C₁- bis C₃-Alkyl-Rest oder C₁ bis C₃-Alkoxy-Rest ist, der vorzugsweise linear ist und der eine endständige radikalisch polymerisierbare Gruppe, vorzugweise Vinyl, Acrylat, Methacrylat, tragen kann, wobei
- R¹: H oder ein vorzugsweise linearer C₁- bis C₃-Alkyl-Rest ist.

Wenn Ar ein Phenyl-Rest und m = 1 ist, befindet sich der Rest R vorzugsweise in para-Stellung zur yl-Stelle, wenn m = 2 oder 3 ist, befinden sich die Reste R vorzugsweise in ortho- und para-Stellung zur yl-Stelle. Die bevorzugten und besonders bevorzugten Bedeutungen der einzelnen Variablen können jeweils unabhängig voneinander gewählt werden.

Ganz besonders bevorzugt sind Verbindungen der Formel (I), in denen Ar ein Phenylrest ist, der m-fach durch R substituiert ist. m ist dabei vorzugsweise 1-3, besonders bevorzugt 1, und R ist vorzugsweise eine Elektronendonorgruppe, insbesondere eine Alkoxygruppe.

Erfindungsgemäß sind Verbindungen, der Formel (I) bevorzugt, die ein Absorptionsmaximum bei 400 nm bis 700 nm aufweisen, besonders bevorzugt 400 bis 550 nm, wie z.B. Tetrabenzoylgermanium oder Tetra(4-methoxybenzoyl)germanium. Das Absorptionsspektrum der Verbindungen der Formel (I) kann durch die Wahl der Gruppe R gezielt eingestellt werden. Beispielsweise bewirken NO₂- oder CN-Substituenten eine bathochrome Verschiebung des Absorptionsspektrums, d.h., dass Verbindungen, in denen einer oder mehrere der Reste R = CN sind, längerwelliges Licht absorbieren, so dass die Polymerisation durch sichtbares Licht im längerwelligen Bereich ausgelöst werden kann.

Tetraacylgermane der allgemeinen Formel (I) sind aus dem Stand der Technik nicht bekannt und sind mit herkömmlichen Verfahren nicht herstellbar. Diese Verbindungen zeichnen sich durch eine hohe Reaktivität, d.h. eine ausgezeichnete polymerisationsauslösende Wirkung und eine gute Durchhärtungstiefe beim Bestrahlen mit sichtbarem Licht aus. Dies ist nicht nur bei Dentalmaterialien und insbesondere bei dentalen Füllungskompositen von großem Vorteil, sondern auch bei nicht dentalen Anwendungen.

Es wurde überraschenderweise gefunden, dass sich (Arylacyl)ₖ(alkyl)₄₋ₖgermane der Formel I' durch die Umsetzung von (Trialkylsilyl)germanen der Formel (R'₃Si)ₖGeR"₄₋ₖ (II) in Gegenwart einer Base und eines Arylacylhalogenids (III) herstellen lassen. 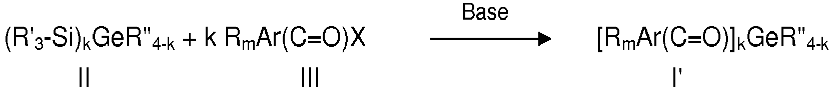 wobei:
- R': eine Alkylgruppe mit 1 bis 6, vorzugsweise 1 bis 4 C-Atomen ist, besonders bevorzugt CH₃,
- R": eine Alkylgruppe mit 1 bis 12, vorzugsweise 1 bis 6, besonders bevorzugt 1 bis 4 C-Atomen ist, ganz besonders bevorzugt CH₃, C₂H₅ oder C₄H₉,
- X: F, Cl, Br oder I ist, vorzugsweise F oder Cl,
- k: eine ganze Zahl von 1 bis 4 ist und
- R: die oben angegebene Bedeutung hat.

Im Fall von R' und R" sind in allen Fällen lineare Alkylgruppen bevorzugt.

Als Basen werden vorzugsweise Alkalimetallalkoholate, besonders bevorzugt Kalium-tert.-butylat, Alkalimetallamide, besonders bevorzugt Lithiumdiisopropylamid, oder alkalimetallorganische Verbindungen, besonders bevorzugt n-Butyl-lithium, eingesetzt.

Bevorzugte Arylacylhalogenide der Formel III leiten sich unmittelbar von den bevorzugten und besonders bevorzugten Definitionen der Gruppen Ar und R ab. Beispielhafte Verbindungen hierfür sind:

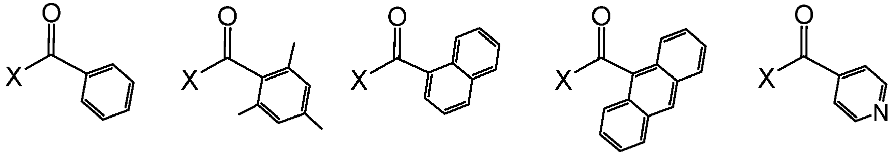

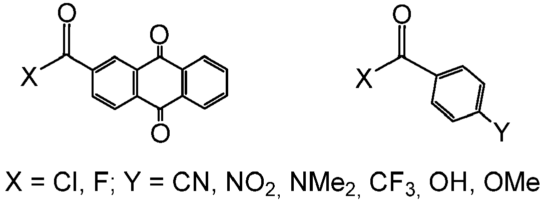

Vorzugsweise wird das Trimethylsilylgerman (R'₃Si)ₖGeR"₄₋ₖ (II) zunächst mit der Base zu (R'₃Si)ₖ₋₁R"₄₋ₖGeM umgesetzt, wobei M ein Metallion, vorzugweise ein Erdalkali- und insbesondere ein Alkalimetallion ist, und anschließend wird (R'₃Si)ₖ₋₁R"₄₋ₖGeM mit dem Acylhalogenid der Formel (III) in eine Verbindung der Formel (R'₃Si)ₖ₋₁R"₄₋ₖGe(C=O)ArRₘ überführt. Auf diese Weise werden die (R'₃Si)-Gruppen der Formel (II) nacheinander gegen -(C=O)ArRₘ Reste ausgetauscht. Die Zwischenprodukte (R'₃Si)ₖ₋₁R"₄₋ₖGeM werden vorzugsweise nicht isoliert.

Mit dem erfindungsgemäßen Verfahren können Acylgermane der Formel (I') in hoher Reinheit und mit guten Ausbeuten hergestellt werden. Ein besonderer Vorteil ist, dass auf aufwendige Schutzgruppentechnik unter Verwendung schwefelhaltiger Schutzgruppen verzichtet werden kann. Schwefelhaltige Verunreinigungen sind nur sehr schwer aus den Produkten zu entfernen, und schon geringe Spuren an schwefelhaltigen Rückständen führen zu einem unangenehmen Geruch des Endprodukts.

Die für die Synthese der Acylgermane der Formel (I') benötigten Ausgangsmaterialien (R'₃Si)ₖGeR"₄₋ₖ (II) können vorzugsweise durch die Umsetzung der entsprechenden Germaniumchloride ClₖGeR"₄₋ₖ (IV) mit einem Trialkylsilylbromid der Formel R'₃SiBr oder vorzugsweise einem Trialkylsilylchlorid der Formel R'₃SiCl (V) hergestellt werden:

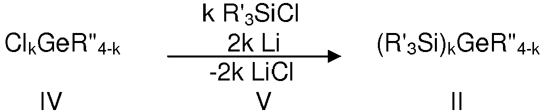

Dazu wird vorzugsweise eine Suspension von fein verteiltem Li (vorzugsweise 1,7 bis 2,2k, besonders bevorzugt 1,85k Äquivalente) in einem passenden Lösungsmittel zuerst mit R'₃SiBr oder bevorzugt R'₃SiCl (vorzugsweise 0,9 bis 1,1k, besonders bevorzugt 0,99k Äquivalente) und anschließend langsam mit einer Lösung des Germaniumchlorids (vorzugsweise 1 Äquivalent) versetzt. Als Lösungsmittel wird dabei jeweils bevorzugt ein Ether, besonders bevorzugt THF eingesetzt. Die verwendete Lösungsmittelmenge beträgt vorzugsweise 20 bis 40 ml/g Li, ganz besonders bevorzugt 30 ml/g Li, bzw. 1 bis 5 ml/g GeCl₄, ganz besonders bevorzugt 2,5 ml/g GeCl₄. Die Reaktionstemperatur beträgt vorzugsweise +30 bis -100°C, besonders bevorzugt -78°C. Die Aufarbeitung des Produktgemisches erfolgt vorzugsweise durch Filtration, bevorzugt über Kieselgur (Celite®), saure Hydrolyse, bevorzugt mit einer Mischung von H₂SO₄/Eis, Phasentrennung und anschließendes Entfernen des Lösungsmittels, vorzugsweise durch Destillation. Das Produkte kann vorteilhaft durch Kristallisation, Sublimation oder Destillation isoliert werden.

Gemäß einer besonders bevorzugten Ausführungsform des erfindungsgemäßen Verfahrens lassen sich Acylgermane der Formel (I') durch die Umsetzung von Trimethylsilylgermanen (Me₃Si)ₖGeR"₄₋ₖ (II') mit Kalium-tert.-butylat (KOtBu) und die anschließende Reaktion mit einem Acylhalogenid (III) (X = F oder Cl) herstellen:

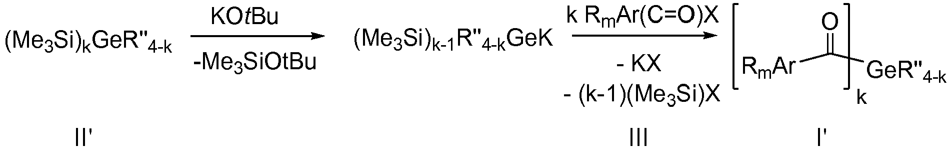

Zur Bildung von (Me₃Si)ₖ₋₁R"₄₋ₖGeK werden vorzugsweise zunächst (Me₃Si)ₖGeR"₄₋ₖ (bevorzugt 1 Äquivalent) und KOtBu (bevorzugt 0,9 bis 4 Äquivalente, besonders bevorzugt 1,1 Äquivalente) in einem passenden Lösungsmittel gelöst und bis zum vollständigen Ablauf der Reaktion gerührt. Als Lösungsmittel wird dabei bevorzugt ein Ether, besonders bevorzugt DME (Dimethoxyethan) eingesetzt. Die verwendete Lösungsmittelmenge beträgt vorzugsweise 10 bis 60 ml/g KOtBu, besonders bevorzugt 20 ml/g KOtBu. Die Reaktionstemperatur beträgt vorzugsweise +80 bis -30°C, besonders bevorzugt +25°C, die Reaktionszeit vorzugsweise 0,5 bis 3 Stunden, besonders bevorzugt 1 Stunde.

Danach wird dann das Acylhalogenid (III) (vorzugsweise 1,0 bis 1,5 Äquivalente) zugegeben und bis zum vollständigen Ablauf der Reaktion gerührt, um das Acylgerman der Formel (I') zu erhalten. Das Acylhalogenid (III) kann dabei sowohl in Substanz als auch in Lösung eingesetzt werden, wobei die Lösungsmittelmenge vorzugsweise 0 bis 200 ml, besonders bevorzugt 100 ml/mmol Acylhalogenid beträgt. Als Lösungsmittel wird vorzugsweise ein Ether, besonders bevorzugt Diethylether, verwendet. Die Reaktionstemperatur beträgt dabei vorzugsweise +30 bis -100°C, ganz besonders bevorzugt -78°C. Die Reaktionszeit beträgt vorzugsweise 0,5 bis 48 Stunden, besonders bevorzugt 24 Stunden. Die Aufarbeitung des Produktgemisches erfolgt bevorzugt durch saure Hydrolyse, vorzugsweise mit einer Mischung von H₂SO₄/Eis, Phasentrennung und Entfernen des Lösungsmittels z.B. durch Destillation. Das Produkt kann durch Säulenchromatographie und durch Kristallisation, bevorzugt nur durch Kristallisation isoliert werden.

Durch die Umsetzung von Mono-, Bis-, Tris- oder Tetra-(trialkylsilyl)germanen der Formel (II) mit Acylhalogeniden der Formel (III) sind auf analoge Weise Monoacyltrialkylgermane, Bisacyldialkylgermane und Trisacylmonoalkylgermane direkt und ohne Schutzgruppentechnik herstellbar.

Tetraacylgermane der Formel (I') mit k = 4 sind durch dieses Verfahren erstmals zugänglich. Hierzu wird auf die oben beschriebene Weise ein Trialkylsilylgerman der Formel (R'₃Si)₄Ge in Gegenwart einer Base mit einem aromatischen Arylhalogenid der Formel (III) umgesetzt. Das Trialkylsilylgerman (R'₃Si)₄Ge kann wie beschreiben durch die Umsetzung von Germaniumtetrachlorid mit R'₃SiCl und metallischem Li hergestellt werden.

Besonders vorteilhaft lassen sich Tetraacylgermane der Formel (I) wie oben beschrieben durch die Umsetzung von Tetrakis(trimethylsilyl)german (Me₃Si)₄Ge mit Kalium-tert.-butylat (KOtBu) und die anschließende Reaktion mit einem Acylhalogenid der Formel (III) (X = F oder Cl) herstellen:

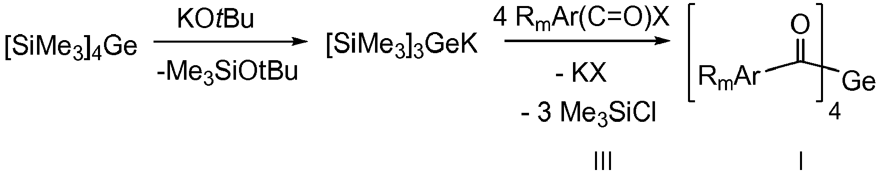

Das Acylhalogenid (III) wird vorzugsweise in einer Menge von 1,0 bis 5 Äquivalenten, besonders bevorzugt 4,1 Äquivalenten zugegeben, und bis zum vollständigen Ablauf der Reaktion gerührt. Das Acylhalogenid kann wie beschreiben sowohl in Substanz als auch in Lösung eingesetzt werden.

Die Tetraacylgermane der allgemeinen Formel (I) und die (Acyl)ₖ(alkyl)₄₋ₖgermane der Formel (I') eignen sich besonders als Photoinitiatoren für die Polymerisation, insbesondere als Initiatoren für die radikalische Polymerisation, Photoaddition und für die Thiol-En-Reaktion (Polyaddition). Es wurde gefunden, dass mit diesen Initiatoren bei Bestrahlung mit Licht eine hohe Durchhärtungstiefe erzielt werden kann, ohne dass die Initiatoren zu Verfärbungen führen. Dies ist bei vielen technischen und besonders medizinischen Werkstoffen von großem Vorteil.

Die Verbindungen der allgemeinen Formeln (I) und (I') eignen sich besonders zur Herstellung von Dentalwerkstoffen, Knochenzementen und ganz besonders von Kontaktlinsen, Intraokularlinsen oder anderen medizinischen Formteilen, wie z.B. von Gehörschalen, Knorpelimplantaten und künstlichen Gewebeteilen.

Die große Durchhärtungstiefe bei der Härtung mit Licht im sichtbaren Wellenlängenbereich ist auch bei technischen Anwendungen ein erheblicher Vorteil. Die Initiatoren der Formeln (I) und (I') eignen sich daher auch für eine Vielzahl von nicht medizinischen Anwendungszwecken, wie zum Beispiel zur Herstellung von Druck- oder Anstrichfarben, Lacken, Adhäsiven, zur Herstellung von Druckplatten, integrierten Schaltkreisen, Photoresists, Lötmasken, Tinten für Farbdrucker, als Materialien für die holographische Datenspeicherung, zur Herstellung von nano-dimensionierten mikroelektromechanischen Elementen, Lichtwellenleitern, Formteilen und zur optischen Herstellung von Informationsträgern. Ein Hauptanwendungsgebiet ist die Verwendung als Photoinitiator bei der stereolithographischen Herstellung von technischen Formteilen, z.B. von Präzisionsformteilen und von keramischen Grünkörpern.

Die erfindungsgemäßen Zusammensetzungen enthalten bezogen auf die Gesamtmasse der Zusammensetzung vorzugsweise 0,001 bis 5 Gew.-%, besonders bevorzugt 0,01 bis 1,0 Gew.-% der Acylgermanium-Verbindung der Formel (I) oder (I'). Neben der Acylgermanium-Verbindung der Formel (I) oder (I') enthalten die Zusammensetzungen vorzugsweise auch ein polymerisierbares Bindemittel. Bevorzugte Bindemittel sind radikalisch und/oder kationisch polymerisierbare Monomere und/oder Prepolymere, besonders bevorzugt radikalisch polymerisierbare Monomere, radikalisch polymerisierbare Prepolymere oder eine Mischung davon.

Als radikalisch polymerisierbare Bindemittel eignen sich besonders mono- oder multifunktionelle (Meth)acrylate oder deren Mischung. Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3 polymerisierbaren Gruppen verstanden.

Diesbezügliche Beispiele sind Methyl-, Ethyl-, Hydroxyethyl-, Butyl-, Benzyl-, Tetrahydrofurfuryl- oder Isobornyl(meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, sowie Gylcerindi- und -trimethacrylat, 1,4-Butandioldi(meth)acrylat, 1,10-Decandioldi(meth)acrylat oder 1,12-Dodecandioldi(meth)acrylat. Zusammensetzungen, die mindestens ein radikalisch polymerisierbares Monomer mit 2 oder mehr, vorzugsweise 2 bis 3 radikalisch polymerisierbaren Gruppen enthalten, sind besonders bevorzugt. Mehrfach funktionelle Monomere haben vernetzende Eigenschaften.

Als radikalisch polymerisierbare Bindemittel lassen sich auch hydrolysestabile Verdünnermonomere wie hydrolysestabile Mono-(meth)acrylate, z.B. Mesitylmethacrylat oder 2-(Alkoyxymethyl)acrylsäuren, z.B. 2-(Ethoxymethyl)acrylsäure, 2-(Hydroxymethyl)acrylsäure, N-mono- oder -disubstitiuierte Acrylamide, wie z.B. N-Ethylacrylamid, N,N-Dimethacrylamid, N-(2-Hydroxyethyl)acrylamid oder N-Methyl-N-(2-hydroxyethyl)acrylamid, bzw. N-monosubstituierte Methacrylamide, wie z.B. N-Ethylmethacrylamid oder N-(2-Hydroxyethyl)methacrylamid sowie außerdem N-Vinylpyrrolidon oder Allylether einsetzen. Bevorzugte Beispiele für hydrolysestabile Vernetzermonomere sind Urethane aus 2-(Hydroxymethyl)acrylsäure und Diisocyanten, wie 2,2,4-Trimethylhexamethylendiisocyanat oder Isophorondiisocyanat, vernetzende Pyrrolidone, wie z.B. 1,6-Bis(3-vinyl-2-pyrrolidonyl)-hexan, oder kommerziell zugängliche Bisacrylamide wie Methylen- oder Ethylenbisacrylamid, bzw. Bis(meth)-acrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechlorid synthetisiert werden können.

Weiterhin lassen sich als radikalisch polymerisierbare Bindemittel auch bekannte schrumpfungsarme radikalisch ringöffnend polymerisierbare Monomere wie z.B. mono- oder multifunktionelle Vinylcyclopropane bzw. bicyclische Cyclopropanderivate (vgl. DE 196 16 183 C2 bzw. EP 1 413 569 A1) oder cyclische Allylsulfide (vgl. US 6,043,361 oder US 6,344,556) einsetzen, die darüber hinaus auch in Kombination mit den voranstehend aufgeführten Di(meth)acrylat-Vernetzern verwendet werden können. Geeignete ringöffnend polymerisierbare Monomere sind solche Vinylcyclopropane, wie 1,1-Di(ethoxycarbonyl)- oder 1,1-Di(methoxycarbonyl)-2-vinylcyclopropan oder die Ester der 1-Ethoxycarbonyl- oder 1-Methoxycarbonyl-2-vinylcyclopropancarbonsäure mit Ethylenglycol, 1,1,1-Trimethylolpropan, 1,4-Cyclohexandiol oder Resorcin. Geeignete bicyclische Cyclopropanderivate sind 2-(Bicyclo[3.1.0]hex-1-yl)acrylsäuremethyl- oder -ethylester oder deren Disubstitutionsprodukte in 3-Stellung wie (3,3-Bis(ethoxycarbonyl)bicyclo[3.1.0]hex-1-yl)-acrylsäuremethyl- oder -ethylester. Geeignete cyclische Allylsulfide sind die Additionsprodukte von 2-(Hydroxymethyl)-6-methylen-1,4-dithiepan oder 7-Hydroxy-3-methylen-1,5-dithiacylooctan mit 2,2,4-Trimethylhexamethylen-1,6-diisocyanat oder einem asymmetrischen Hexamethylendiisocyanat-Trimeren (z.B. Desmodur® VP LS 2294 der Bayer AG).

Bevorzugt sind auch Formulierungen auf Basis von Vinylestern, Vinylcarbonaten und Vinylcarbamaten als radikalisch polymerisierbare Monomere. Ausserdem können als Monomere auch Styrol, Styrolderivate oder Divinylbenzol, ungesättigte Polyesterharze sowie Allylverbindungen oder radikalisch polymerisierbare Polysiloxane, die aus geeigneten Methacrylsilanen, wie z.B. 3-(Methacryloyloxy)propyltrimethoxysilan, hergestellt werden können und z.B. in der DE 199 03 177 C2 beschrieben sind, eingesetzt werden.

Weiterhin lassen sich als radikalisch polymerisierbare Bindemittel auch Mischungen der voranstehend genannten Monomere mit radikalisch polymerisierbaren, säuregruppenhaltigen Monomeren verwenden, die auch als Haftmonomere bezeichnet werden. Bevorzugte säuregruppenhaltige Monomere sind polymerisationsfähige Carbonsäuren, wie Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin oder 4-Vinylbenzoesäure.

Als Haftmonomere eignen sich auch radikalisch polymerisierbare Phosphonsäuremonomere, insbesondere Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxa-butyl]-acrylsäure oder 2-[2-Dihydroxyphosphoryl)-ethoxymethyl]-acrylsäure-ethyl- oder 2,4,6-trimethylphenylester.

Zudem eignen sich als Haftmonomere acide polymerisationsfähige Phosphorsäureester, insbesondere 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritol-pentamethacryloyloxyphosphat, 10-Methacryloyloxydecyl-dihydrogenphosphat, Dipentaerythritol-pentamethacryloyloxyphosphat, Phosphorsäuremono-(1-acryloylpiperidin-4-yl)-ester, 6-(Methacrylamido)hexyldihydrogenphosphat und 1,3-Bis-(N-acryloyl-N-propyl-amino)-propan-2-yl-dihydrogenphosphat.

Darüber hinaus eignen sich polymerisationsfähige Sulfonsäuren als Haftmonomere, insbesondere Vinylsulfonsäure, 4-Vinylphenylsulfonsäure oder 3-(Methacrylamido)propylsulfonsäure.

Als durch Polyaddition härtbare Bindemittel eignen sich besonders Thiol-En-Harze, die Mischungen von mono- oder multifunktionellen Mecaptoverbindungen und di- oder multifunktionellen ungesättigten Monomeren, vor allem Allyl- oder Norbonenverbindungen, enthalten.

Beispiele für mono- oder multifunktionellen Mecaptoverbindungen sind o-, m- bzw. p-Dimercaptobenzol und Ester der Thioglykol- oder der 3-Mercaptopropionsäure von Ethylen-, Propylen- oder Butylenglykol, Hexandiol, Glycerin, Trimethylolpropan oder Pentaerythrit.

Beispiele für di- oder multifunktionelle Allylverbindungen sind Ester von Allylalkohol mit Di- oder Tricarbonsäuren, wie Malon-, Malein-, Glutar-, Bernstein-, Adipin-, Sebacin-, Phthal-, Terephthal- oder Gallussäure sowie mono- oder trifunktionelle Allylether, wie z.B. Diallylether, α,ω-Bis[allyloxy]alkane, Resorcin- oder Hydrochinondiallylether sowie Pyrogalloltriallylether, oder andere Verbindungen wie z.B. 1,3,5-Triallyl-1,3,5-triazin-2,4,6-(1H,3H,5H)-trion, Tetraallylsilan oder Tetraallylorthosilikat.

Beispiele für di- oder multifunktionelle Norbonenverbindungen sind Diels-Alder-Additionsprodukte von Cyclopentadien oder Furan mit di- oder multifunktionellen (Meth)acrylaten, sowie Ester bzw. Urethane von 5-Norbornen-2-methanol oder 5-Norbornen-2-ol mit Di- oder Polycarbonsäuren, wie z.B. Malon-, Malein-, Glutar-, Bernstein-, Adipin-, Sebacin-, Phthal-, Terephthal- oder Gallussäure, mit bzw. Di- oder Polyisocyanaten, wie Hexamethylendiisocyanat bzw. dessen cyclischem Trimer, 2,2,4-Trimethylhexamethylendiisocyanat, Toluylendiisocyanat oder Isophorondiisocyanat.

Neben Acylgermanium-Verbindungen der allgemeinen Formel (I) können die erfindungsgemäßen Zusammensetzungen vorteilhaft zusätzlich auch bekannte Photoinitiatoren (vgl. J.P. Fouassier, J.F. Rabek (Hrsg.), Radiation Curing in Polymer Science and Technology, Vol. II, Elsevier Applied Science, London und New York 1993) für den UV- oder sichtbaren Bereich, wie z.B: Benzoinether, Dialkylbenzilketale, Dialkoxyacetophenone, Acylder Bisacylphosphinoxide, α-Diketone wie 9,10-Phenanthrenchinon, Diacetyl, Furil, Anisil, 4,4'-Dichlorbenzil und 4,4'-Dialkoxybenzil und Campherchinon enthalten.

Ausserdem können die erfindungsgemässen Zusammensetzungen neben den Tetraacylgermanen der allgemeinen Formel (I) und/oder den (Acyl)ₖ(alkyl)₄₋ₖgermanen der Formel I' zur dualen Härtung auch Azoverbindungen, wie 2,2'-Azobis(isobutyronitril) (AIBN) oder Azobis-(4-cyanovaleriansäure), oder Peroxide, wie Dibenzoylperoxid, Dilauroylperoxid, *tert*-Butylperoctoat, *tert-*Butylperbenzoat oder Di-(tert-butyl)-peroxid enthalten. Zur Beschleunigung der Initiierung mittels Peroxiden lassen sich auch Kombinationen mit aromatischen Aminen einsetzen. Redoxsysteme, die sich bereits bewährt haben, sind: Kombinationen aus Benzoylperoxid mit Aminen, wie N,N-Dimethyl-p-toluidin, N,N-Dihydroxyethyl-p-toluidin, p-Dimethylaminobenzoesäureethylester oder strukturverwandte Systeme. Darüber hinaus sind auch Redoxsysteme bestehend aus Peroxiden und solchen Reduktionsmitteln, wie z.B. Ascorbinsäure, Barbiturate oder Sulfinsäuren oder Kombinationen von Hydroperoxiden mit Reduktionsmitteln und katalytischen Metallionen, wie z.B. eine Mischung von Cumolhydroperoxid, einem Thioharnstoffderivat und Kupfer(II)-acetylacetonat, für die duale Aushärtung geeignet.

Erfindungsgemäß sind Zusammensetzungen bevorzugt, die einen oder mehrere Füllstoffe, vorzugsweise organische oder anorganische partikuläre Füllstoffe enthalten. Bevorzugte anorganische partikulären Füllstoffe sind amorphe kugelförmige nanopartikuläre Füllstoffe auf der Basis von Oxiden, wie pyrogene Kieselsäure oder Fällungskieselsäure, ZrO₂ und TiO₂ oder Mischoxide aus SiO₂, ZrO₂ und/oder TiO₂ mit einem mittleren Partikeldurchmesser von 10 bis 200 nm, Minifüllstoffe, wie Quarz-, Glaskeramik- oder Glaspulver mit einer durchschnittlichen Teilchengrösse von 0,2 bis 5 µm sowie röntgenopake Füllstoffe, wie Ytterbiumtrifluorid oder nanopartikuläres Tantal(V)-oxid bzw. Bariumsulfat. Darüber hinaus können auch faserförmige Füllstoffe wie Nanofasern, Glasfasern, Polyamid- oder Kohlenstoff-Fasern eingesetzt werden.

Für nichtdentale Anwendungen kommen neben den oben genannten Materialien außerdem Homo- und/oder Copolymere, vorzugsweise Poly((meth)acrylat)e, Vinylpolymere, vorzugsweise Polystyrol oder Polyvinylacetat, oder Kondensationspolymere, vorzugsweise Polyester, als Füllstoffe in Betracht. Diese Füller werden vorzugsweise als Pulver mit einer mittleren Partikelgröße zwischen 0,5 und 100 µm eingesetzt. Sie sind zum Teil im Monomer löslich.

Außerdem können die erfindungsgemäßen Zusammensetzungen im Bedarfsfalle weitere Additive, wie z.B. Stabilisatoren, UV-Absorber, Farbstoffe oder Pigmente sowie Lösungsmittel, wie z.B. Wasser, Ethanol, Aceton oder Ethylacetat, oder Gleitmittel enthalten.

Die erfindungsgemäßen Werkstoffe enthalten vorzugsweise:
(a) 0,001 bis 5 Gew.-% Tetraacylgerman(e) der allgemeinen Formel (I),
(b) 10 bis 99,9 Gew.-% radikalisch polymerisierbares Bindemittel,
(c) 0 bis 85 Gew.-% Füllstoff und ggf.
(d) 0 bis 70 Gew.-% Additiv(e).

Alle Prozentangaben beziehen sich, wenn nicht anders angegeben, auf die Gesamtmasse des Materials.

Werkstoffe, die sich besonders als dentale Zemente eignen, enthalten bevorzugt:
(a) 0,001 bis 5 Gew.-% Tetraacylgerman(e) der allgemeinen Formel (I),
(b) 10 bis 50 Gew.-% radikalisch polymerisierbares Bindemittel,
(c) 40 bis 70 Gew.-% Füllstoff und
(d) 0 bis 5 Gew.-% Additiv.

Werkstoffe, die sich besonders als dentale Komposite eignen, enthalten bevorzugt:
(a) 0,001 bis 5 Gew.-% Tetraacylgerman(e) der allgemeinen Formel (I),
(b) 10 bis 40 Gew.-% radikalisch polymerisierbares Bindemittel,
(c) 50 bis 70 Gew.-% Füllstoff und
(d) 0 bis 5 Gew.-% Additiv(e).

Werkstoffe, die sich besonders als dentale Beschichtungsmaterialien eignen, enthalten bevorzugt:
(a) 0,001 bis 5 Gew.-% Tetraacylgerman(e) der allgemeinen Formel (I),
(b) 20 bis 99,9 Gew.-% radikalisch polymerisierbares Bindemittel,
(c) 0 bis 20 Gew.-% nanopartikuläre Füllstoffe und
(d) 0,01 bis 2 Gew.-% Additiv(e),
(e) 0 bis 70 Gew.-% Lösungsmittel.

Werkstoffe, die sich besonders als dentale Adhäsive eignen, enthalten bevorzugt:
(a) 0,001 bis 5 Gew.-% Tetraacylgerman(e) der allgemeinen Formel (I),
(b) 20 bis 98,99 Gew.-% radikalisch polymerisierbares Bindemittel,
(c) 0 bis 20 Gew.-% nanopartikuläre Füllstoffe
(d) 0,01 bis 2 Gew.-% Additiv,
(e) 0 bis 50 Gew.-% Lösungsmittel und
(f) 1 bis 20 Gew.-% radikalisch polymerisierbares Haftmonomer.

Werkstoffe für dentale Prothesen oder chirurgische Formkörper enthalten bevorzugt:
(a) 0,001 bis 5 Gew.-% Tetraacylgerman(e) der allgemeinen Formel (I),
(b) 30 bis 99,9 Gew.-% radikalisch polymerisierbares Bindemittel,
(c) 0 bis 60 Gew.-% Füllstoff(e) und ggf.
(d) 0 bis 30 Gew.-% Additiv(e).

Werkstoffe für Kunststoffformteile enthalten bevorzugt:
(a) 0,001 bis 5 Gew.-% Tetraacylgerman(e) der allgemeinen Formel (I),
(b) 30 bis 99,9 Gew.-% radikalisch polymerisierbares Bindemittel,
(c) 0 bis 60 Gew.-% Füllstoff und ggf.
(d) 0 bis 15 Gew.-% Additiv(e).

Werkstoffe für keramische Grünkörper enthalten bevorzugt:
(a) 0,001 bis 5 Gew.-% Tetraacylgerman(e) der allgemeinen Formel (I),
(b) 0 bis 40 Gew.-% radikalisch polymerisierbares Bindemittel,
(c) 40 bis 90 Gew.-% Füllstoff und ggf.
(d) 0 bis 20 Gew.-% Additiv(e).

Die erfindungsgemäßen Materialien, die Tetraacylgermane der allgemeinen Formel (I) als Photoinitiator enthalten, lassen sich zur Herstellung von Photopolymerisaten, Kompositen, Zementen, Beschichtungsmaterialien, Primern oder Adhäsiven einsetzen. Sie sind besonders geeignet für Anwendungen im medizinischen Bereich, vor allem zur Herstellung von Dentalmaterialien, wie Füllungskompositen, Befestigungszementen, Adhäsiven, Prothesenmaterialien, Verblendmaterialien, Kronen oder Inlays oder von Beschichtungen.

Die Dentalwerkstoffe eignen sich primär zur intraoralen Anwendung durch den Zahnarzt zur Restauration geschädigter Zähne, d.h. zur therapeutischen Anwendung, z.B. als dentale Zemente, Füllungskomposite und Verblendmaterialien. Sie können aber auch extraoral eingesetzt werden, beispielsweise bei der Herstellung oder Reparatur von Dentalrestaurationen, wie Prothesen, künstlichen Zähnen, Inlays, Onlays, Kronen und Brücken.

Weiterhin eignen sich die erfindungsgemäßen Materialien zur medizinischen Anwendung in der Chirurgie, z.B. in der Geweberegeneration, zur Herstellung von Gehörhilfen oder in der Augenheilkunde zur Herstellung von Intraokularlinsen oder Kontaktlinsen.

Bei technischen Anwendungen lassen sich die Tetraacylgermane der allgemeinen Formel (I) als Photoinitiator in der Stereolithographie oder im 3D-Druck zur Herstellung von Formkörpern, Prototypen oder Grünkörpern, im Beschichtungsbereich oder in der Mikroelektronik z.B. in der Photoresist-Technologie einsetzen.

Die Erfindung wird im Folgenden anhand von Beispielen näher erläutert.

### Beispiel 1:

### Synthese von Tetrabenzoylgerman (TBGe)

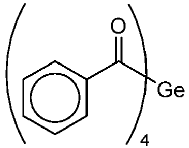

### a) Synthese von Tetrakis(trimethylsilyl)german[(Me₃Si)₄Ge]

Es wurden 10,00 g (1.4 mol) Lithium in einem Kolben versehen mit Tropftrichter und Druckausgleicher vorgelegt und mit 300 mL trockenem THF versetzt. Bei -78°C wurde Trimethylchlorsilan (95 ml, 0,75 mol) zügig zugetropft und für 10 min. gerührt. Anschließend wurde bei -78°C Germaniumtetrachlorid (21 ml, 0,19 mol, 1:5 verdünnt in THF) sehr langsam zugetropft (ca. 2h). Nach beendeter Zugabe wurde die Reaktionslösung auf Raumtemperatur erwärmt und für weitere 12 Stunden gerührt. Zur Aufarbeitung wurde das Reaktionsgemisch zuerst über Celite filtriert und anschließend auf 1 M H₂SO₄/Eis geschüttet. Nach Phasentrennung im Tropftrichter wurde die wässrige Phase 3 mal mit Diethylether extrahiert, die vereinigten organischen Phasen über wasserfreiem Na₂SO₄ getrocknet, filtriert und das Lösungsmittel am Rotavapor entfernt. Zur Reinigung wurde das Rohprodukt sublimiert (p < 5 mbar; T > 150 °C). Die Ausbeute betrug nach Sublimation 26,8 g (Me₃Si)₄Ge (42 %) .

NMR-Spektroskopie: ¹H(CDCl₃) : δ [ppm] = 0.24 (s, Si(C*H*₃)₃). ²⁹Si (CDCl₃): δ [ppm] = -5.33 (*Si*Me₃).

### b) Synthese von Tetrabenzoylgerman (TBGe)

3,00 g (8,21 mmol; 1,00 eq.) Tetrakistrimethylsilylgerman und 1,01 g KOtBu (9,03 mmol; 1,1 eq.) wurden in einem Schlenkkolben eingewogen und in 20 ml Ethylenglycoldimethylether (DME) gelöst. Die Reaktion war beendet, wenn die Reaktionslösung eine klare gelbe bis orange Farbe aufwies. Nach ca. einer Stunde wurden 4,18 g (33,66 mmol, 4,1 eq.) Benzoylfluorid mittels Spritze zugegeben. Die Reaktionslösung wurde schwarz und nach vollständiger Zugabe orange. Anschließend wurde die Reaktionslösung bei Raumtemperatur über Nacht gerührt. Nach wässriger Aufarbeitung mit 3 %iger H₂SO₄ wurden die Phasen getrennt und die wässrige Phase 3 mal mit Diethylether extrahiert. Die gesammelten organischen Phasen wurden über wasserfreiem Natriumsulfat getrocknet und die flüchtigen Bestandteile am Rotationsverdampfer entfernt. Das erhaltene Rohprodukt wurde aus Aceton umkristallisiert und 1,70 g reines Tetrabenzoylgerman (42 %) als kristalliner, gelber Feststoff (Schmelzpunkt: 82,5-83,0 °C) erhalten.
NMR-Spektroskopie: ¹H (CDCl₃): δ [ppm] = 7,99-7,96 (m, 2H, Aryl-H), 6,84-6,82 (m, 3H, Aryl-H). ¹³C (CDCl₃): δ [ppm] = 222,01 (GeCOPh), 140,57 (Aryl-Cl), 133,81 (Aryl-C2), 129,15 (Aryl-C3) 128,77 (Aryl-C4).
UV-VIS- Spektroskopie: λ [nm] (ε[L mol⁻¹ cm⁻¹]) = 403 (1240), 419sh (1050).
IR- Spektroskopie: v [cm⁻¹] = 1639, 1617 (m, vC=O); 1590, 1574, 1444 (m, VC=C); 880,762, 673 (s, δC-H).

### Beispiel 2:

### Synthese von Tetrakis(2,4, 6-trimethylbenzoyl)german (TMGe)

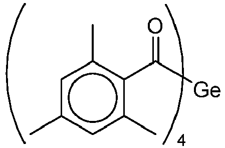

2,77 g (7,66 mmol; 1,00 eq.) (Me₃Si)₄Ge und 0.94 g KOtBu (8,4 mmol; 1,1 eq.) wurden in einem Schlenkgefäss eingewogen und in 15 ml DME gelöst. Die Reaktion war beendet, wenn die Reaktionslösung eine klare gelbe bis orange Farbe aufwies. Nach ca. einer Stunde wurde die erhaltene Lösung langsam zu einer auf -78 °C gekühlten Lösung von 1,66 g (0,91 mmol, 1,2 eq.) 2,4,6-Trimethylbenzoylchlorid in 80 ml Diethylether getropft und die erhaltene Mischung über Nacht bei Raumtemperatur gerührt. Nach wässriger Aufarbeitung mit 3 %iger H₂SO₄ wurden die Phasen getrennt und die wässrige Phase 3 mal mit Diethylether extrahiert. Die gesammelten organischen Phasen wurden über wasserfreiem Natriumsulfat getrocknet und die flüchtigen Bestandteile am Rotationsverdampfer entfernt. Das gebildete Rohprodukt mit einer Masse von 3.85 g enthielt 36 % Tetraacylgermanium- und 64 % Monoacylgermanium-Verbindung und wurde durch Säulenchromatographie über Kieselgel (Gradient: Heptan,Toluol) getrennt. Anschließend wurde aus Aceton umkristallisiert, und es wurden 1,58 g (24 %) Tetrakis(2,4,6-trimethylbenzoyl)german als kristalliner, gelber Feststoff (Schmelzpunkt: 198-199°C) erhalten.
NMR-Spektroskopie: ¹H (CDCl₃): δ [ppm] = 6,57 (s, 2H, Aryl-H), 2,24 (s, 3H, para-C*H*₃), 2,06 (s, 6H, ortho-C*H*₃). ¹³C (CDCl₃): δ
[ppm] = 233,40 (Ge*C*OMes), 141,60 (Aryl-*C*1), 139,26 (Aryl-*C*2), 132,88 (Aryl-*C*3) 128,53 (Aryl-*C*4), 21,15 (para-*C*H₃), 19,13 (ortho-*C*H₃).
UV-VIS- Spektroskopie: λ [nm] (ε[L mol⁻¹ cm⁻¹]) = 288 (17428), 376 (1475).
IR- Spektroskopie: v [cm⁻¹] = 2917 (w, vₐₛCH₃); 1639, 1608 (m, VC=O); 1202 (m, δₐₛCH₃); 833, 609 (m, ρCH₃).

### Beispiel 3:

### Herstellung von lichthärtbaren Harzen unter Verwendung von Tetrabenzoylgerman (TBGe) bzw. Tetrakis(2,4,6-trimethylbenzoyl)german (TMGe) aus Beispiel 1 und 2

Aus einer Mischung (Angaben in Masse-%) der Dimethacrylate Bis-GMA (Additionsprodukt aus Methacrylsäure und Bisphenol-A-diglycidylether)), UDMA (Additionsprodukt aus 2-Hydroxyethylmethacrylat und 2,2,4-Trimethylhexamethylendiisocyanat) und D₃MA (Decandiol-1,10-dimethacrylat) und den Ge-Initiatoren Tetrabenzoylgerman (TBGe), Tetrakis(2,4,6-trimethylbenzoyl)-german (TMGe) und Dibenzoyldiethylgerman (DBEGe, als Referenz) (Tabelle 1) wurden verschiedene lichthärtende Harzsysteme hergestellt. Dabei enthalten die Harzsysteme **R1** und **R4** (0,29 mmol/100 g) bzw. **R2, R3** und **R5** (0,59 mmol/100 g) die gleiche molare Menge an Photoinitiator.

**Tabelle 1: Zusammensetzung der Harze R1 bis R5**

| **Komponente/Harz** | **R1** | **R2** | **R3** | **R4*** | **R5*** |
|---|---|---|---|---|---|
| TBGe | 0,14 | 0,29 | - | - | - |
| TMGe | - | - | 0,39 | - | - |
| DBEGe | - | - | - | 0,10 | 0,20 |
| Bis-GMA | 42,10 | 42,10 | 42,10 | 42,10 | 42,10 |
| UDMA | 37,46 | 37,31 | 37,21 | 37,50 | 37,40 |
| D₃MA | 20,30 | 20,30 | 20,30 | 20,30 | 20,30 |

| | | | | | |
|---|---|---|---|---|---|
| * Vergleichsbeispiel | | | | | |

Von den Materialien wurden Prüfkörper präpariert, die 2 mal 3 Minuten mit einer dentalen Lichtquelle (Spectramat®, Ivoclar Vivadent AG) bestrahlt und damit ausgehärtet wurden. Nach der ISO-Norm ISO-4049 (Dentistry - Polymer-based filling, restorative and luting materials) erfolgte die Bestimmung der Biegefestigkeit und des Biege-E-Moduls nach 24 h Lagerung der Prüfkörper bei Raumtemperatur (RT) oder nach 24 h Lagerung in Wasser bei 37 °C (WL) (Tabelle 2).

**Tabelle 2: Biegefestigkeit (BF, MPa) und Biege-E-Modul (BM, GPa) der polymerisierten Harze R1 bis R5**

| | **R1** | **R2** | **R3** | **R4*** | **R5*** |
|---|---|---|---|---|---|
| BF, RT | 72,7±3,5 | 81,7±9,5 | 81,5±5,5 | 58,5±2,3 | 79,7±7,5 |
| BF, WL | 98,2±8,7 | 115,2±11,2 | 101,8±6,1 | 75,3±3,0 | 96,4±8,1 |
| BM, RT | 1,59±0,12 | 2,25±0,22 | 1,89±0,21 | 1,15±0,07 | 1,76±0,19 |
| BM, WL | 1,15±0,20 | 2,48±0,10 | 2,37±0,12 | 1,54±0,11 | 2,19±0,20 |

| | | | | | |
|---|---|---|---|---|---|
| * Vergleichsbeispiel | | | | | |

Die Ergebnisse in Tabelle 2 belegen, dass die Harze **R1** und **R3** mit dem erfindungsgemäßen Tetra(benzoyl)german TBGe als Photoinitiator im Vergleich zu den Referenzharzen **R4** und **R5** auf Basis des bekannten Di(benzoyl)germans DBEGe bei gleicher molarer Konzentration der Photoinitiatoren (vergleiche **R1** mit **R4** bzw. **R2** mit **R5**) zu Photopolymerisaten mit einer verbesserten Festigkeit und höherem E-Modul führen.

### Beispiel 4:

### Herstellung von lichthärtbaren Harzen unter Verwendung von Tetrabenzoylgerman (TBGe) bzw. Tetrakis (2,4,6-trimethylbenzoyl)-german (TMGe) aus Beispiel 1 und 2

Aus den Harzen **R1** bis **R5** aus Beispiel 3 wurden die Kompositpasten **K1** bis **K5** mittels eines Walzenstuhles (Modell "Exakt", Exakt Apparatebau, Norderstedt) hergestellt. Dabei wurden jeweils 36,44 Gew.-% der Harze **R1** bis **R5** mit 52,22 Gew.-% an silanisiertem Glasfüller GM 27884 (1,0 pm, Schott), 4,02 Gew.-% an silanisiertem Glasfüller GM G018-056 (1,0 pm, Schott), 4,02 Gew.-% an dem silanisiertem SiO₂-ZrO₂-Mischoxid Sphärosil (Transparent Materials, USA) 0,80 Gew.-% der silanisierten pyrogenen Kieselsäure OX-50 (Degussa) und 2,50 Gew.-% Ytterbiumtrifluorid YbF₃ (Sukgyung, Südkorea) gefüllt. Von den Pasten wurden analog Beispiel 3 Prüfkörper präpariert, ausgehärtet und die Biegefestigkeit und der E-Modul bestimmt (Tabelle 3).

**Tabelle 3: Biegefestigkeit (BF, MPa) und Biege-E-Modul (BM, GPa) der polymerisierten Kompositpasten K1 bis K5**

| | **K1** | **K2** | **K3** | **K4*** | **K5*** |
|---|---|---|---|---|---|
| BF, RT | 96,5±9,2 | 125,4±7,7 | 114,8±4,6 | 92,4±6,4 | 112±6,9 |
| BF, WL | 117,4±8,7 | 129, 7 ± 9, 9 | 133,8±4,8 | 101,9±9,0 | 123,3±3,5 |
| BM, RT | 5,51±0,33 | 7,13±0,40 | 6,73±0,37 | 4,99±0,39 | 6,20±0,32 |
| BM, WL | 6,16±0.46 | 7,68±0.87 | 7,36±0.62 | 5,45±0.64 | 6,59±0.34 |

| | | | | | |
|---|---|---|---|---|---|
| * Vergleichsbeispiel | | | | | |

Die Ergebnisse in Tabelle 3 belegen, dass die Kompositpasten **K1** und **K3** mit dem erfindungsgemäßen Tetra(benzoyl)german TBGe als Photoinitiator im Vergleich zu den Referenzpasten **K4** bzw. **K5** auf Basis des bekannten Di(benzoyl)germans DBEGe bei gleicher molarer Konzentration der Photoinitiatoren (vergleiche **K1** mit **K4** bzw. **K2** mit **K5**) nach der Aushärtung zu Kompositen mit einer verbesserten Festigkeit und höherem E-Modul führen.

## Patentansprüche

1. Acylgermanium-Verbindung gemäß der allgemeinen Formel (I),
[RₘAr-(C=O)-]₄-Ge (I)
in der die Variablen die folgenden Bedeutungen haben:
Ar ein mono- oder polyzyklischer Kohlenwasserstoffrest mit 6 bis 18 Ringkohlenstoffatomen, der m-fach durch die Gruppe R substituiert sein kann und der ein oder mehrere ringständige Heteroatome enthalten kann, wobei
m eine ganze Zahl von 0 bis 6 ist und nicht größer als die Anzahl der substituierbaren Wasserstoffatomen in Ar sein kann,
R Halogen, NR¹₂, OH, OSiR²₃, (C=O)R³, CN, NO₂, CF₃, COOR⁴, oder ein C₁- bis C₂₀-Alkyl-, -Alkenyl-, -Alkoxy- oder -Alkenoxy-Rest ist, der linear, verzweigt oder zyklisch sein kann, der durch ein oder mehrere O-Atome unterbrochen sein kann und der eine radikalisch polymerisierbare Gruppe enthalten kann, oder =0, wobei
R¹-R³ unabhängig voneinander jeweils H oder ein linearer oder verzweigter C₁- bis C₁₂-Alkyl-Rest sind und
R⁴ H, ein linearer oder verzweigter C₁- bis C₁₂-Alkyl-Rest oder SiR⁵₃ ist, wobei
R⁵ ein linearer oder verzweigter C₁- bis C₁₀-Alkyl-Rest.

2. Acylgermanium-Verbindung nach Anspruch 1, in der die Variablen die folgenden Bedeutungen haben:
Ar ein aromatischer C₆-C₁₀-Rest, der m-fach durch R substituiert sein kann, wobei
m eine ganze Zahl von 1 bis 3 ist und
R Cl, NR¹₂, OSiR²₃, (C=O)R³, CN, NO₂, CF₃, COOR⁴, oder ein C₁- bis C₁₀-Alkyl-, -Alkenyl-, -Alkoxy- oder -Alkenoxy-Rest ist, der linear, verzweigt oder zyklisch sein kann, der durch ein oder mehrere O-Atome unterbrochen sein kann, und der eine radikalisch polymerisierbare Gruppe, vorzugweise Vinyl, Methacrylat, (Meth)acrylamid oder N-Alkylacrylamid enthalten kann, wobei die radikalisch polymerisierbare Gruppe bei nichtzyklischen Resten vorzugsweise endständige ist, wobei
R¹-R³ unabhängig voneinander jeweils H oder ein linearer oder verzweigter C₁- bis C₈-Alkyl-Rest sind und
R⁴ H, ein linearer oder verzweigter C₁- bis C₈-Alkyl-Rest oder SiR⁵₃ ist und
R⁵ ein linearer oder verzweigter C₁- bis C₅-Alkyl-Rest ist.

3. Acylgermanium-Verbindung nach Anspruch 1 oder 2, in der die Variablen die folgenden Bedeutungen haben:
Ar ein Phenyl-Rest, Pyridyl-Rest, Naphthyl-Rest, Anthryl-Rest, Anthrachinonyl-Rest, der m-fach durch R substituiert sein kann, wobei
m eine ganze Zahl von 1 bis 3 ist und
R NR¹₂, CN, NO₂, CF₃, ein C₁- bis C₃-Alkyl-Rest oder C₁-bis C₃-Alkoxy-Rest ist, der vorzugsweise linear ist und der eine endständige radikalisch polymerisierbare Gruppe, vorzugweise Vinyl, Acrylat, Methacrylat, tragen kann, wobei
R¹ H oder ein vorzugsweise linearer C₁- bis C₃-Alkyl-Rest ist.

4. Zusammensetzung, die bezogen auf ihre Gesamtmasse 0,001 bis 5 Gew.-% einer Acylgermanium-Verbindung der Formel (I) gemäß einem der Ansprüche 1 bis 3 und mindestens ein polymerisierbares Bindemittel enthält.

5. Zusammensetzung nach Anspruch 4, die als polymerisierbares Bindemittel mindestens ein radikalisch polymerisierbare Monomer und/oder Prepolymer enthält.

6. Zusammensetzung nach Anspruch 5, die als Bindemittel mindestens ein mono- oder multifunktionelles (Meth)acrylat oder eine Mischung davon enthält.

7. Zusammensetzung nach einem der Ansprüche 4 bis 6, die 0,001 - 5 Gew.-% Acylgermanium-Verbindung der Formel (I), 10 - 99,9 Gew.-% polymerisierbares Bindemittel, 0 - 85 Gew.-% Füllstoff enthält, jeweils bezogen auf die Gesamtmasse der Zusammensetzung.

8. Verwendung eines Acylgermans gemäß Formel (I) als Initiator für die radikalische Polymerisation.

9. Verfahren zur Herstellung von (Arylacyl)ₖ(alkyl)₄₋ₖgermanen der Formel I' (k = 1 bis 4), bei dem man ein (Trialkylsilyl)german der Formel (R'₂Si)ₖGeR"₄₋ₖ (II) in Gegenwart einer Base mit einem Arylacylhalogenid (III) zu I' umsetzt, 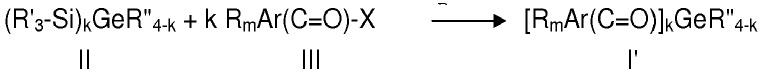 wobei:
R' eine Alkylgruppe mit 1 bis 6 C-Atomen ist,
R" eine Alkylgruppe mit 1 bis 12 C-Atomen ist,
X F, Cl, Br oder I ist,
k eine ganze Zahl von 1 bis 4 ist und
R die in Anspruch 1, 2 oder 3 angegebene Bedeutung hat.

10. Verfahren nach Anspruch 9, bei dem man als Base ein Alkalimetallalkoholat, ein Alkalimetallamid oder eine alkalimetallorganische Verbindung einsetzt.

11. Verfahren nach Anspruch 9 oder 10, bei dem man als Arylacylhalogenid der Formel III eine der folgenden Verbindungen einsetzt:

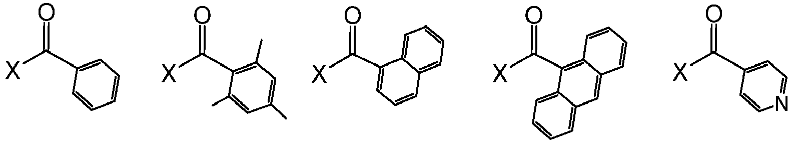

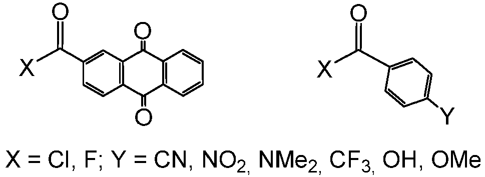

12. Verfahren nach einem der Ansprüche 9 bis 11, bei dem man das Trimethylsilylgerman (R'₃Si)ₖGeR"₄₋ₖ (II) zunächst mit der Base zu (R'₃Si)ₖ₋₁R"₄₋ₖGeM umgesetzt, wobei M ein Metallion ist, und man anschließend (R'₃Si)ₖ₋₁R"₄₋ₖGeM mit dem Acylhalogenid der Formel (III) in eine Verbindung der Formel (I) überführt.

13. Verfahren nach einem der Ansprüche 9 bis 12, bei dem man die Verbindung der Formel (II) herstellt, indem man ein Germaniumchlorid der Formel ClₖGeR"₄₋ₖ (IV) mit einem Trialkylsilylchlorid der Formel R'₃SiCl (V) umsetzt:

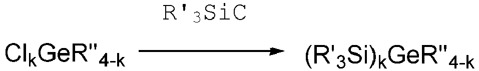

14. Verfahren gemäß einem der Ansprüche 9 bis 13, bei dem man zunächst ein Trimethylsilylgerman der Formel (Me₃Si)ₖGeR"₄₋ₖ (II') mit Kalium-tert.-butylat (KOtBu) und anschließend mit einem Acylhalogenid der Formel (III) (X = F oder Cl) umsetzt.

15. Verfahren nach einem der Ansprüche 9 bis 14, bei dem man ein Tetra(trialkylsilyl)german der Formel (R'₃Si)₄Ge in Anwesenheit einer Base mit einem Acylhalogenid der Formel (III) umsetzt, um eine Verbindung der Formel (I) zu erhalten.

## Claims

1. Acylgermanium compound according to the general Formula (I),
[RₘAr-(C=O)-]₄-Ge (I)
in which the variables have the following meanings:
Ar a mono- or polycyclic hydrocarbon residue containing 6 to 18 ring-carbon atoms which may be substituted m times by the R group and which may comprise one or more heteroatoms in the ring, wherein
m is an integer from 0 to 6 and cannot be greater than the number of substitutable hydrogen atoms in Ar,
R is halogen, NR¹₂, OH, OSiR²₃, (C=O)R³, CN, NO₂, CF₃, COOR⁴, or a C₁ to C₂₀-alkyl, -alkenyl, -alkoxy or -alkenoxy group, which may be linear, branched or cyclic, which may be interrupted by one or more O atoms and which may comprise a radically polymerisable group, or =O, wherein
R¹-R³ independently of each other are in each case H or a linear or branched C₁ to C₁₂-alkyl group and
R⁴ is H, a linear or branched C₁ to C₁₂-alkyl group or SiR⁵₃, wherein
R⁵ is a linear or branched C₁ to C₁₀-alkyl group.

2. Acylgermanium compound according to claim 1, in which the variables have the following meanings:
Ar an aromatic C₆-C₁₀ residue, which may be substituted m times by R, wherein
m is an integer from 1 to 3 and
R is Cl, NR¹₂, OSiR²₃, (C=O)R³, CN, NO₂, CF₃, COOR⁴, or a C₁ to C₁₀-alkyl, -alkenyl, -alkoxy or -alkenoxy group, which may be linear, branched or cyclic, which may be interrupted by one or more O atoms, and which may comprise a radically polymerisable group, preferably a vinyl, methacrylate, (meth)acrylamide or *N*-alkylacrylamide, wherein the radically polymerisable group in the case of acyclic groups is preferably terminal, wherein
R¹-R³ independently of each other are in each case H or a linear or branched C₁ to C₈-alkyl group and
R⁴ is H, a linear or branched C₁ to C₈-alkyl group or SiR⁵₃ and
R⁵ is a linear or branched C₁ to C₅-alkyl group.

3. Acylgermanium compound according to claim 1 or 2, in which the variables have the following meanings:
Ar a phenyl group, pyridyl group, naphthyl group, anthryl group, anthraquinonyl group, which may be substituted m times by R, wherein
m is an integer from 1 to 3 and
R is NR¹₂, CN, NO₂, CF₃, a C₁ to C₃-alkyl group or C₁ to C₃-alkoxy group, which is preferably linear and which may bear a terminal radically polymerisable group, preferably vinyl, acrylate, methacrylate, wherein
R¹ is H or a preferably linear C₁ to C₃-alkyl group.

4. Composition which, relative to its total mass, comprises 0.001 to 5 wt % of an acylgermanium compound of Formula (I) according to one of claims 1 to 3, and at least one polymerisable binder.

5. Composition according to claim 4 which comprises at least one radically polymerisable monomer and/or prepolymer as the polymerisable binder.

6. Composition according to claim 5, which comprises at least one mono- or multifunctional (meth)acrylate or a mixture thereof as the binder.

7. Composition according to one of claims 4 to 6, which comprises 0.001 - 5 wt % acylgermanium compound of Formula (I),
10 to 99.9 wt % polymerisable binder,
0 to 85 wt % filler,
in each case relative to the total mass of the composition.

8. Use of an acylgermane according to Formula (I) as the initiator for radical polymerisation.

9. Process for the preparation of (arylacyl)ₖ(alkyl)₄₋ₖgermanes of Formula I' (k = 1 to 4), in which a (trialkylsilyl)germane of Formula (R'₃Si)ₖGeR"₄₋ₖ (II) is reacted in the presence of a base with an arylacyl halide (III) to afford I', 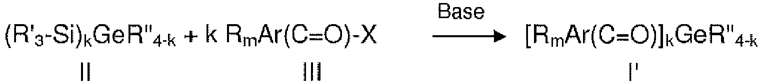 wherein:
R' is an alkyl group containing 1 to 6 C atoms,
R" is an alkyl group containing 1 to 12 C atoms,
X is F, Cl, Br or I,
k is an integer from 1 to 4 and
R has the meaning given in claim 1, 2 or 3.

10. Process according to claim 9, in which an alkali metal alcoholate, an alkali metal amide or an organometallic alkali metal compound is used as the base.

11. Process according to claim 9 or 10, in which one of the following compounds is used as the arylacyl halide of Formula III:

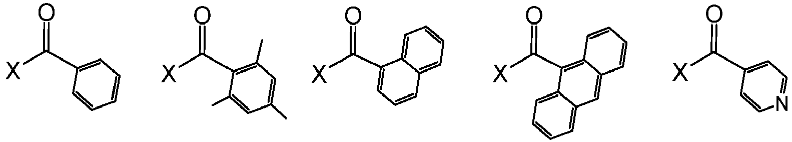

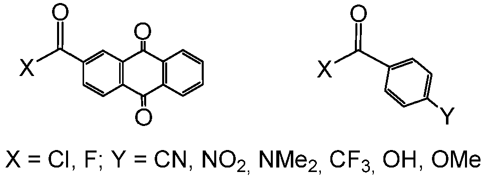

12. Process according to one of claims 9 to 11, in which the trimethylsilylgermane (R'₃Si)ₖGeR"₄₋ₖ (II) is first reacted with the base to afford (R'₃Si)ₖ₋₁R"₄₋ₖGeM, wherein M is a metal ion, and the (R'₃Si)ₖ₋₁R"₄₋ₖGeM is then converted with the acyl halide of Formula (III) into a compound of Formula (I).

13. Process according to one of claims 9 to 12, in which the compound of Formula (II) is prepared by reacting a germanium chloride of Formula ClₖGeR"₄₋ₖ (IV) with a trialkylsilyl chloride of Formula R'₃SiCl (V):

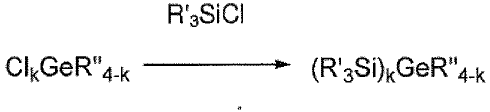

14. Process according to one of claims 9 to 13, in which a trimethylsilylgermane of Formula (Me₃Si)ₖGeR"₄₋ₖ (II') is initially reacted with potassium tert-butylate (KO*t*Bu) and then with an acyl halide of Formula (III) (X = F or CI).

15. Process according to one of claims 9 to 14, in which a tetra(trialkylsilyl)germane of Formula (R'₃Si)₄Ge is reacted in the presence of a base with an acyl halide of Formula (III), in order to obtain a compound of Formula (I).

## Revendications

1. Composé acylgermanium selon la formule générale (I),
[RₘAr-(C=O)-]₄-Ge (I)
dans laquelle les variables ont les significations suivantes :
Ar représente un radical hydrocarboné mono- ou polycyclique ayant de 6 à 18 atomes de carbone formant le cycle, qui peut être m fois substitué par le groupe R et qui peut contenir un ou plusieurs hétéroatomes dans le cycle,
m étant un nombre entier valant de 0 à 6 et ne pouvant pas être supérieur au nombre des atomes d'hydrogènes susceptibles d'être remplacés dans Ar,
R représente un atome d'halogène, NR¹₂, OH, OSiR²₃, (C=O)R³, CN, NO₂, CF₃, COOR⁴, ou un radical alkyle, alcényle, alcoxy ou alcénoxy en C₁-C₂₀, qui peut être linéaire, ramifié ou cyclique, qui peut être interrompu par un ou plusieurs atomes d'oxygène et qui peut contenir un groupe apte à la polymérisation radicalaire, ou =O,
R¹-R³ étant chacun indépendamment l'un de l'autre un atome d'hydrogène ou un radical alkyle en C₁-C₁₂ linéaire ou ramifié et
R⁴ étant un atome d'hydrogène, un radical alkyle en C₁-C₁₂ linéaire ou ramifié ou SiR⁵₃,
R⁵ représentant un radical alkyle en C₁-C₁₀ linéaire ou ramifié.

2. Composé acylgermanium selon la revendication 1, dans lequel les variables ont les significations suivantes :
Ar représente un radical aromatique en C₆-C₁₀, qui peut être m fois substitué par le groupe R,
m étant un nombre entier valant de 1 à 3 et
R représente Cl, NR¹₂, OSiR²₃, (C=O)R³, CN, NO₂, CF₃, COOR⁴, ou un radical alkyle, alcényle, alcoxy ou alcénoxy en C₁-C₁₀, qui peut être linéaire, ramifié ou cyclique, qui peut être interrompu par un ou plusieurs atomes d'oxygène et qui peut contenir un groupe apte à la polymérisation radicalaire, de préférence un groupe vinyle, méthacrylate, (méth)acrylamide ou N-alkylacrylamide, le groupe apte à la polymérisation radicalaire, lorsque les radicaux ne sont pas cycliques, étant de préférence en bout de chaîne,
R¹-R³ étant chacun indépendamment l'un de l'autre un atome d'hydrogène ou un radical alkyle en C₁-C₈ linéaire ou ramifié et
R⁴ étant un atome d'hydrogène, un radical alkyle en C₁-C₈ linéaire ou ramifié ou SiR⁵₃ et
R⁵ représentant un radical alkyle en C₁-C₅ linéaire ou ramifié.

3. Composé acylgermanium selon la revendication 1 ou 2, dans lequel les variables ont les significations suivantes :
Ar représente un radical phényle, radical pyridyle, radical naphtyle, radical anthryle, radical anthraquinonyle, qui peut être m fois substitué par un groupe R,
m étant un nombre entier valant de 1 à 3 et
R représente NR¹₂, CN, NO₂, CF₃, un radical alkyle en C₁-C₃ ou alcoxy en C₁-C₃, qui est de préférence linéaire et qui peut porter un groupe apte à la polymérisation radicalaire en bout de chaîne, de préférence un groupe vinyle, acrylate, méthacrylate,
R¹ étant un atome d'hydrogène ou un radical alkyle en C₁-C₃ de préférence linéaire.

4. Composition, qui contient par rapport à sa masse totale 0,001 à 5 % en poids d'un composé acylgermanium de formule (I) selon l'une quelconque des revendications 1 à 3 et au moins un liant polymérisable.

5. Composition selon la revendication 4, qui contient comme liant polymérisable au moins un monomère et/ou un prépolymère, polymérisable(s) par voie radicalaire.

6. Composition selon la revendication 5, qui contient comme liant au moins un (méth)acrylate mono- ou polyfonctionnel ou un mélange de tels (méth)acrylates.

7. Composition selon l'une quelconque des revendications 4 à 6, qui contient 0,001 - 5 % en poids de composé acylgermanium de formule (I), 10 - 99,9 % en poids de liant polymérisable, 0 - 85 % en poids de charge, chaque fois par rapport à la masse totale de la composition.

8. Utilisation d'un composé acylgermanium conforme à la formule (I) en tant qu'amorceur pour la polymérisation radicalaire.

9. Procédé pour la préparation de composés (arylacyl)ₖ(alkyl)₄₋ₖgermanium de formule I' (k = 1 à 4), dans lequel on fait réagir un composé (trialkylsilyl)germanium de formule (R'₃Si)ₖGeR"₄₋ₖ (II), en présence d'une base, avec un halogénure d'arylacyle (III) pour aboutir à un composé I', 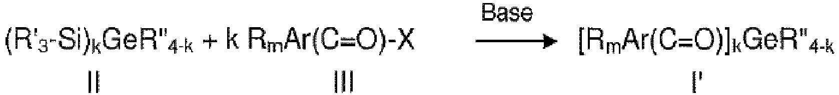 où :
R' est un groupe alkyle ayant de 1 à 6 atomes de carbone,
R" est un groupe alkyle ayant de 1 à 12 atomes de carbone,
X est F, Cl, Br ou I,
k est un nombre entier valant de 1 à 4 et
R a la signification indiquée dans la revendication 1, 2 ou 3.

10. Procédé selon la revendication 9, dans lequel on utilise comme base un alcoolate de métal alcalin, un amidure de métal alcalin ou un composé organométallique de métal alcalin.

11. Procédé selon la revendication 9 ou 10, dans lequel on utilise comme halogénure d'arylacyle de formule III l'un des composés suivants :

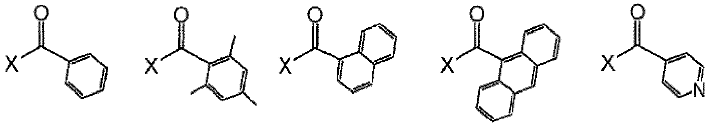

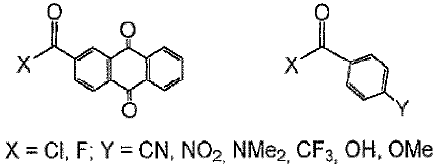

12. Procédé selon l'une quelconque des revendications 9 à 11, dans lequel on fait d'abord réagir le composé triméthylsilylgermanium (R'₃Si)ₖGeR"₄₋ₖ (II) avec la base pour aboutir à un composé (R'₃Si)ₖ₋₁R"₄₋ₖGeM, M étant un ion métallique, et ensuite on convertit le composé (R'₃Si)ₖ₋₁R"₄₋ₖGeM avec l'halogénure d'acyle de formule (III) en un composé de formule (I).

13. Procédé selon l'une quelconque des revendications 9 à 12, dans lequel on prépare le composé de formule (II) en faisant réagir un chlorure de germanium de formule ClₖGeR"₄₋ₖ (IV) avec un chlorure de trialkylsilyle de formule R'₃SiCl (V) :

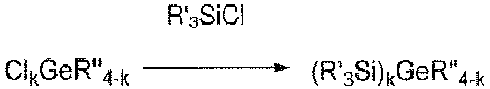

14. Procédé selon l'une quelconque des revendications 9 à 13, dans lequel on fait réagir un composé triméthylsilylgermanium de formule (Me₃Si)ₖGeR"₄₋ₖ (II') d'abord avec du tert-butylate de potassium (KOtBu) et ensuite avec un halogénure d'acyle de formule (III) (X = F ou Cl).

15. Procédé selon l'une quelconque des revendications 9 à 14, dans lequel on fait réagir un composé tétra(trialkylsilyl)germanium de formule (R'₃Si)₄Ge, en présence d'une base, avec un halogénure d'acyle de formule (III), pour obtenir un composé de formule (I).
